# EUROPEAN PATENT SPECIFICATION

(11) **EP 3 738 951 B1**
(45) Date of publication and mention of the grant of the patent: **14.09.2022**
(21) Application number: 18941896.5
(22) Date of filing: 27.11.2018
(51) Int. Cl.: C08F 2/38, C07D 207/267

(54) **USE OF A POLYMERIZATION INHIBITOR FOR N-VINYL PYRROLIDONE MONOMER**
VERWENDUNG VON POLYMERISATIONSINHIBITOR FÜR N-VINYLPYRROLIDON-MONOMER
UTILISATION D'UN INHIBITEUR DE POLYMÉRISATION POUR MONOMÈRE DE N-VINYLPYRROLIDONE

(43) Date of publication of application: 18.11.2020
(73) Proprietor: Boai NKY Medical Holdings Ltd., 454450 Jiaozuo, Henan (CN)
(72) Inventor: SUN, Xudong, Jiaozuo, Henan 454450 (CN); NIU, Lei, Jiaozuo, Henan 454450 (CN); ZHANG, Senlin, Jiaozuo, Henan 454450 (CN); LI, Haipeng, Jiaozuo, Henan 454450 (CN)
(74) Representative: Sebastian, Jens
(86) International application number: PCT/CN2018/117618
(87) International publication number: WO 2020/107197

(56) References cited:
- WO-A1-2004/103965
- WO-A1-2006/109869
- CN-B- 102 267 942

## Description

### BACKGROUND OF THE INVENTION

### Field of the Invention

The present invention belongs to the technical field of chemical reagents, in particular to the use of a polymerization inhibitor of N-vinyl pyrrolidone monomers.

### Background Art

N-vinyl pyrrolidone is abbreviated as NVP, which is a vinylation product of 2- pyrrolidone. Its molecular structure consists of vinyl group and five-membered cyclic lactam groups. NVP is a colorless transparent liquid, easily soluble in water, alcohol, ether and other organic solvents, with a melting point of 13.5°C and the structural formula as follows.

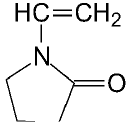

N-vinyl pyrrolidone is active in property due to five-membered cyclic carbonyl group thereof in that N-vinyl pyrrolidone polymerizes under weak acid conditions or initiators, and the self-polymerization thereof will be accelerated under the conditions of heating, vibration, air/oxygen contact, etc. It is reported that when N-vinyl pyrrolidone is sealed and preserved at 17~23°C for a long time, the self-polymer thereof will also be generated. Therefore, 0.1% sodium hydroxide, ammonia water, low molecular organic amine and the like are usually added as polymerization inhibitors in the commercial process to avoid the generation of self-polymer and extend the shelf life.

Although the addition of sodium hydroxide and ammonia water can inhibit the rapid generation of self-polymer, they also speed up the deepening of the color of N-vinyl pyrrolidone from colorless to light yellow, yellow and even brown. The low molecular organic ammonia is mainly composed of N,N'-bis(1-methylpropyl)-1,4-phenylenediamine, a 10ppm dosage of which can achieve excellent polymerization inhibition effect, but still cannot avoid color deepening from colorless to pink, red and brown red. Meanwhile N,N'-bis(1-methylpropyl)-1,4-phenylenediamine itself is a brown hypertoxic substance. Other common inhibitors used for preservation, such as antioxidant phenol/quinone inhibitors like 2,6-di-tert-butyl-4-methylphenol, tert-butylcatechol, benzoquinone and the like also have common problems of color deepening and high toxicity. In addition, most of high temperature inhibitors need to be removed by distillation or other methods before use.

CN 102267942 B discloses a preparation of 3-Amino-4-methylpyridine in the presence of an acid-binding agent, which could be e.g. potassium carbonate or potassium acetate. However, CN 102267942 B does not disclose other uses of potassium carbonate and potassium acetate.

### SUMMARY OF THE INVENTION

In view of the above situation, an object of the present invention is to provide the use of a polymerization inhibitor of N-vinyl pyrrolidone monomers. According to the characteristic of N-vinyl pyrrolidone as being easy to self-polymerize under the condition of weak acidity, a kind of weak acid potassium salt are provided, which is added directly in proportion to inhibit the self-polymerization of N-vinyl pyrrolidone monomers. The inhibitor can extend the shelf life and reduce the chromaticity and toxicity of monomers to a great extent. The first aspect of the present invention provides the use of a polymerization inhibitor of N-vinyl pyrrolidone monomers, which is selected from an inorganic weak acid potassium salt and/or an organic acid potassium salt.

In the present invention, the polymerization inhibitor may be conventional inorganic weak acid potassium salt, organic acid potassium salt, or a mixture thereof. Preferably, the polymerization inhibitor is one or more selected from the group consisting of potassium carbonate, potassium acetate, potassium propionate, potassium oxalate, potassium citrate and potassium tartrate.

The second aspect of the present invention provides the use of the above polymerization inhibitor in preservation or transportation of N-vinyl pyrrolidone monomers.

Specifically, a polymerization inhibitor is added into the N-vinyl pyrrolidone monomers which are then sealed and preserved under the protection of inert gas. That is, the polymerization inhibitor is added into the preservation and transportation container of the N-vinyl pyrrolidone monomers to be preserved or transported directly to prevent self-polymerization and hence extend the shelf life.

In the present invention, the addition amount of the polymerization inhibitor may be 0.005-0.2% by mass with respect to the mass of the N-vinyl pyrrolidone monomers. Preferably, the addition amount of the polymerization inhibitor is 0.01-0.15% by mass with respect to the mass of the N-vinyl pyrrolidone monomers. More preferably, the addition amount of the polymerization inhibitor is 0.02 to 0.12% by mass with respect to the mass of the N-vinyl pyrrolidone monomers.

The polymerization inhibitor of the present invention can be used in a wide temperature range, all of which has obvious polymerization inhibition effect. Preferably, the preservation temperature of the N-vinyl pyrrolidone monomers added with the polymerization inhibitor is 120°C or less.

In order to ensure better polymerization inhibition effect, it is more preferred that the preservation temperature of the N-vinyl pyrrolidone monomers added with the polymerization inhibitor is 15-40°C.

Compared with the prior art, the present invention has the following advantages:
The polymerization inhibitor does not affect the normal use of the N-vinyl pyrrolidone monomers after being added. The polymerization inhibitor has small influence on chromaticity, which renders the color of the monomers almost unchanged, namely being colorless to light yellow. The adding of the polymerization inhibitor is mainly used for preservation and transportation of the N-vinyl pyrrolidone monomers, which can prevent self-polymerization of the N-vinyl pyrrolidone monomers during preservation and transportation and thus extend the shelf life, and can reduce the risk of serious discoloration of the N-vinyl pyrrolidone. When the polymerization inhibitor is used below 40°C, the shelf life of the N-vinyl pyrrolidone monomers can be extended by more than 2 times. In addition, after the polymerization inhibitor is added, the N-vinyl pyrrolidone can have a solution polymerization directly without removing the polymerization inhibitor, and the color of the N-vinyl pyrrolidone monomers, the polymerization rate during solution polymerization and the chromaticity of the polymerization solution are not affected. The dosage of polymerization inhibitor is small, while a wide temperature range is available for polymerization inhibition. The polymerization inhibitor is a white solid, does not have or produce other colors, and the toxicity thereof is extremely small.

### DETAILED DESCRIPTION OF THE INVENTION

The technical solution of the present invention will be clearly and completely described hereinafter with reference to the examples of the present invention. Obviously, the described examples are only a part of the examples of the present invention, but not all of the examples.

Examples 1 to 7 are used to illustrate the inhibitor of the N-vinyl pyrrolidone monomers of the present invention and use thereof.

### Example 1

A group of brown reagent bottles with the same capacity were filled with 100±0.5g of newly produced N-vinyl pyrrolidone monomers respectively, 0.10±0.002g of potassium carbonate was respectively added, and then the bottles were sealed after air was removed by introducing bubbled nitrogen. These bottles were shaken evenly and placed in a 120°C oven. Take a bottle out every 10 minutes from the oven to test the self-polymerization. Self-polymer was detected at the 90th minute but the sample was colorless and transparent liquid. The specific results were shown in Table 1.

### Example 2

A group of brown reagent bottles with the same capacity were filled with 100±0.5g of newly produced N-vinyl pyrrolidone monomers respectively, 0.0250±0.0005g of potassium acetate was respectively added, and then the bottles were sealed after air was removed by introducing bubbled nitrogen. These bottles were shaken evenly and placed in a 120°C oven. Take a bottle out every 20 minutes from the oven to test the self-polymerization. Self-polymer was detected at the 300th minute but the sample was still colorless and transparent liquid. The specific results were shown in Table 1.

### Example 3

A group of brown reagent bottles with the same capacity were filled with 100±0.5g of newly produced N-vinyl pyrrolidone monomers respectively, 0.0250±0.0005g of potassium acetate was respectively added, and then the bottles were sealed after air was removed by introducing bubbled nitrogen. These bottles were shaken evenly and placed in a 40°C oven. Take a bottle out every 10 days from the oven to test the self-polymerization. Self-polymer was detected at the 190th day but the sample was still light yellow transparent liquid. The specific results were shown in Table 2.

### Example 4

A group of brown reagent bottles with the same capacity were filled with 200±0.5g of newly produced N-vinyl pyrrolidone monomers respectively, 0.10±0.005g of potassium propionate was respectively added, and then the bottles were sealed after air was removed by introducing bubbled nitrogen. These bottles were shaken evenly and placed in a 40°C oven. Take out a bottle every 10 days from the oven to test the self-polymerization. Self-polymer was detected at the 150th day but the sample was still colorless and transparent liquid with a chromaticity <50Haze. The specific results were shown in Table 2.

### Example 5

A group of brown reagent bottles with the same capacity were filled with 100±0.5g of newly produced N-vinyl pyrrolidone monomers respectively, 0.0250±0.0005g of potassium acetate was respectively added, and then the bottles were sealed after air was removed by introducing bubbled nitrogen. These bottles were shaken evenly and placed in a 25°C stability test box (simulating room temperature). Take out a bottle every 30 days (one month) from the box to test the self-polymerization. Self-polymerization was detected at the 10th month (not detected at the 9th month), but the sample was light yellow transparent liquid with a chromaticity of 50Haze. The specific results were shown in Table 3.

### Example 6

A group of brown reagent bottles with the same capacity were filled with 100±0.5g of newly-produced N-vinyl pyrrolidone monomers respectively, 0.050±0.005g of potassium acetate was respectively added, and then the bottles were sealed after air was removed by introducing bubbled nitrogen. These bottles were shaken evenly and placed in a 25°C stability test box (simulating room temperature). Take a bottle out every 30 days (one month) from the box to test the self-polymerization. Self-polymer was detected at the 10th month, but the sample was a light yellow transparent liquid with a chromaticity of 125Haze. The specific results were shown in Table 3.

### Example 7

A group of brown reagent bottles with the same capacity were filled with 200±0.5g of newly-produced N-vinyl pyrrolidone monomers respectively, 0.050±0.005g of potassium acetate and 0.050±0.005g of potassium citrate were respectively added at the same time, and then the bottles were sealed after air was removed by introducing bubbled nitrogen. These bottles were shaken evenly and placed in a 25°C stability test box (simulating room temperature). Take a bottle out every 30 days (one month) from the box to test the self-polymerization. Self-polymer was detected at the 10th month, but the sample was a light yellow transparent liquid with a chromaticity of 100Haze. The specific results were shown in Table 3.

### Control Example 1

Taking the same experimental condition as in Example 1, a group of brown reagent bottles were filled with pure N-vinyl pyrrolidone, then were sealed after air was removed by introducing nitrogen, which were used as a blank control group in which the self-polymerization was detected. In the blank control group, the self-polymerization was detected at the 50th minute, and the sample with self-polymer was colorless and transparent liquid. The specific results were shown in Table 1.

### Control Example 2

Taking the same experimental conditions as in Example 3, a group of brown reagent bottles were filled with pure N-vinyl pyrrolidone, and then were sealed after air was removed by introducing nitrogen, On the 10th day of heating test, self-polymerization has taken place, but the viscosity was relatively small, and the sample was colorless liquid. The specific results were shown in Table 2.

### Control Example 3

A group of brown reagent bottles with the same capacity were filled with 100±0.5g of newly produced N-vinyl pyrrolidone monomers respectively, then 0.10±0.01g of sodium hydroxide was respectively added, and then the bottles were sealed after air was removed by introducing bubbled nitrogen. These bottles were shaken evenly and placed in a 40°C oven. Take a bottle out every 10 days from the oven to test the self-polymerization. Self-polymer was detected at the 50th day, a sample of which is yellow transparent liquid with a chromaticity of 400Haze. The specific results were shown in Table 2.

### Control Example 4

A group of brown reagent bottles with the same capacity were filled with 100±0.5g of newly produced N-vinyl pyrrolidone monomers respectively, 0.10±0.01g of sodium hydroxide was respectively added, and then the bottles were sealed after air was removed by introducing bubbled nitrogen. These bottles were shaken evenly and placed in a stability test box at 25°C. Take a bottle out every 30 days from the oven to test the self-polymerization. Self-polymer was detected at the 7th month (180~210 days) but the sample was yellow transparent liquid with a chromaticity of 250Haze. The specific results were shown in Table 3.

### Control Example 5

A group of brown reagent bottles with the same capacity were filled with 100±0.5g of newly-produced N-vinyl pyrrolidone monomers respectively, bubbled nitrogen was introduced to remove air, and 0.38mL 26.5% ammonia water was added respectively, then the bottles were sealed. These bottles were shaken evenly and placed in a stability test box at 25°C. Take a bottle out every 30 days from the oven to test the self-polymerization. Self-polymer was detected at the 4th month (90~120 days), but the sample was colorless and transparent liquid with a chromaticity <50Haze. The specific results were shown in Table 3.

### Usage Example

The N-vinyl pyrrolidone monomers (from Example 5, that is added with 0.025% potassium acetate and preserved at room temperature for 10 months) were used to synthesiz PVP according to the current PVP K30 polymerization process with a solvent of purified water. After polymerization, a K value of 31.5 was detected, and the polymerization solution was colorless and transparent.

The following tables 1, 2 and 3 record the corresponding data of examples and control examples at 120°C, 40°C and 25°C, respectively.

**Table 1**

| Items | Polymerization inhibitor | Dosage | Time of self-polymerization detected | Color |
|---|---|---|---|---|
| Example 1 | Potassium carbonate | 0.1% | The 90th minute | colorless |
| Example 2 | Potassium acetate | 0.025% | The 300th minute | colorless |
| Control example 1 | -- | -- | The 50th minute | colorless |

**Table 2**

| Items | Polymerization inhibitor | Dosage | Time of self-polymerization detected | Monomer chromaticity |
|---|---|---|---|---|
| Example 3 | Potassium acetate | 0.025% | The 190th day | 50Haze |
| Example 4 | Potassium propionate | 0.1% | The 150th day | <50Haze |
| Control example 2 | -- | -- | The 10th day | <50Haze |
| Control example 3 | Sodium hydroxide | 0.1% | The 50th day | 400Haze |

**Table 3**

| Items | Polymerization inhibitor | Dosage | Time of self-polymerization detected | Monomer chromaticity |
|---|---|---|---|---|
| Example 5 | Potassium acetate | 0.025% | The 10th month | 50Haze |
| Example 6 | Potassium acetate | 0.05% | The 10th month | 125Haze |
| Example 7 | Potassium acetate + potassium citrate | 0.05%+0.05% | The 10th month | 100Haze |
| Control example 4 | Sodium hydroxide | 0.1% | The 7th month | 250Haze |
| Control example 5 | Ammonia | 0.1% | The 4th month | <50Haze |

From the data in Tables 1 to 3, it can be seen that the polymerization inhibitor of the present invention can effectively inhibit the self-polymerization of N-vinyl pyrrolidone monomers, with less dosage, high efficiency, wide temperature range of polymerization inhibition effect, and better effect within the preferred condition range. In addition, as can be seen from the usage example, the polymerization inhibitor does not substantially affect the color of the N-vinyl pyrrolidone monomers, the polymerization rate during solution polymerization, and the chromaticity of the polymerization solution after use.

Examples of the present invention have been described hereinbefore, and the above description is exemplary, not exhaustive, and the present invention is not limited to the disclosed examples.

## Claims

1. Use of polymerization inhibitor in preservation or transportation of N-vinyl pyrrolidone monomers, wherein the polymerization inhibitor is selected from inorganic weak acid potassium salt and/or organic acid potassium salt.

2. The use according to claim 1, wherein the polymerization inhibitor is one or more selected from the group consisting of potassium carbonate, potassium acetate, potassium propionate, potassium oxalate, potassium citrate and potassium tartrate.

3. The use according to claim 1, wherein a polymerization inhibitor is added into the N-vinyl pyrrolidone monomers, and then seal and preservation are carried out under the protection of inert gas.

4. The use according to claim 3, wherein the addition amount of the polymerization inhibitor is 0.005-0.2% by mass with respect to the mass of the N-vinyl pyrrolidone monomers.

5. The use according to claim 4, wherein the addition amount of the polymerization inhibitor is 0.01-0.15% by mass with respect to the mass of the N-vinyl pyrrolidone monomers.

6. The use according to claim 3, wherein the preservation temperature is 120°C or less.

7. The use according to claim 6, wherein the preservation temperature is 15-40°C.

## Patentansprüche

1. Verwendung von Polymerisationsinhibitor bei der Konservierung oder dem Transport von N-Vinylpyrrolidon-Monomeren, wobei der Polymerisationsinhibitor ausgewählt ist aus anorganischem, schwach saurem Kaliumsalz und/oder organischem saurem Kaliumsalz.

2. Verwendung nach Anspruch 1, wobei der Polymerisationsinhibitor einer oder mehrere ist, der bzw. die ausgewählt ist bzw. sind aus der Gruppe, die besteht aus Kaliumcarbonat, Kaliumacetat, Kaliumpropionat, Kaliumoxalat, Kaliumcitrat und Kaliumtartrat.

3. Verwendung nach Anspruch 1, wobei ein Polymerisationsinhibitor den N-Vinylpyrrolidon-Monomeren beigemengt wird und dann Dichtung und Konservierung unter dem Schutz von Inertgas durchgeführt werden.

4. Verwendung nach Anspruch 3, wobei die Beimengungsmenge des Polymerisationsinhibitors 0,005 bis 0,2 Ma%, bezogen auf die Masse der N-Vinylpyrrolidon-Monomere, beträgt.

5. Verwendung nach Anspruch 4, wobei die Beimengungsmenge des Polymerisationsinhibitors 0,01 bis 0,15 Ma%, bezogen auf die Masse der N-Vinylpyrrolidon-Monomere, beträgt.

6. Verwendung nach Anspruch 3, wobei die Konservierungstemperatur 120 °C oder niedriger beträgt.

7. Verwendung nach Anspruch 6, wobei die Konservierungstemperatur 15 bis 40 °C beträgt

## Revendications

1. Utilisation d'un inhibiteur de polymérisation dans la conservation ou le transport de monomères de N-vinylpyrrolidone, dans laquelle l'inhibiteur de polymérisation est choisi parmi un sel potassique d'acide inorganique faible et/ou un sel potassique d'acide organique.

2. Utilisation selon la revendication 1, dans laquelle l'inhibiteur de polymérisation est un ou plusieurs choisis dans le groupe constitué par le carbonate de potassium, l'acétate de potassium, le propionate de potassium, l'oxalate de potassium, le citrate de potassium et le tartrate de potassium.

3. Utilisation selon la revendication 1, dans laquelle un inhibiteur de polymérisation est ajouté dans les monomères de N-vinylpyrrolidone, et ensuite une étanchéité et une conservation sont effectuées sous la protection d'un gaz inerte.

4. Utilisation selon la revendication 3, dans laquelle la quantité ajoutée de l'inhibiteur de polymérisation est de 0,005 à 0,2 % en masse par rapport à la masse des monomères de N-vinylpyrrolidone.

5. Utilisation selon la revendication 4, dans laquelle la quantité ajoutée de l'inhibiteur de polymérisation est de 0,01 à 0,15 % en masse par rapport à la masse des monomères de N-vinylpyrrolidone.

6. Utilisation selon la revendication 3, dans laquelle la température de conservation est de 120°C ou moins.

7. Utilisation selon la revendication 6, dans laquelle la température conservation est de 15 à 40°C.
